# FASCICULE DE BREVET EUROPEEN

(11) **EP 1 216 023 B1**
(45) Date de publication et mention de la délivrance du brevet: **20.04.2005**
(21) Numéro de dépôt: 99946239.3
(22) Date de dépôt: 27.09.1999
(51) Int. Cl.: A61K 7/48, A61K 7/06

(54) **COMPOSITION COSMETIQUE A BASE DE COMPOSES ORGANIQUES DU SILICIUM COMPORTANT AU MOINS UNE FONCTION A EFFET COSMETIQUE**
KOSMETISCHE ZUSAMMENSETZUNG AUF BASIS VON MINDESTENS EINE GRUPPE MIT KOSMETISCHEM EFFEKT ENTHALTENDEN ORGANO-SILIZIUM VERBINDUNGEN
COSMETIC COMPOSITION BASED ON ORGANIC SILICON COMPOUNDS COMPRISING AT LEAST A FUNCTION WITH COSMETIC EFFECT

(43) Date de publication de la demande: 26.06.2002
(73) Titulaire: L'OREAL, 75008 Paris (FR)
(72) Inventeur: SAMAIN, Henri, F-91570 Bievres (FR); ROLLAT-CORVOL, Isabelle, F-75017 Paris (FR); JEANNE ROSE, Valérie, F-75019 Paris (FR); SANCHEZ, Clément, F-91190 Gif-sur-Yvette (FR)
(74) Mandataire: Casalonga, Axel
(86) Numéro de dépôt international: PCT/FR1999/002291
(87) Numéro de publication internationale: WO 2001/022932

(56) Documents cités:
- EP-A- 0 655 453
- EP-A- 1 023 889
- FR-A- 2 725 208
- US-A- 5 750 092

## Description

La présente invention concerne d'une manière générale des compositions cosmétiques aqueuses, en particulier pour le traitement des cheveux, comportant des composés organiques du silicium solubles dans l'eau, peu ou pas polymérisés, ayant deux fonctions non-hydrolysables dont une au moins a un effet cosmétique et deux fonctions hydrolysables par molécule.

Il est habituel d'essayer de traiter les cheveux de façon à améliorer leurs propriétés (démêlage, couleur, douceur). Les différents traitements mis au point permettent d'obtenir de bonnes performances mais, en général, le bénéfice obtenu n'est pas retrouvé lorsque les cheveux sont soumis à un traitement de lavage.

Dans le cas de la coloration, les nombreux travaux ont permis de mettre au point des traitements donnant des performances, colorantes en l'occurence, relativement résistante aux lavages (coloration dite d'oxydation). Cependant, les utilisateurs restent déçus de la résistance aux lavages de leurs produits. De plus, les performances de résistance atteintes à ce jour ont été obtenues au dépend de l'intégrité des cheveux, dans la mesure où l'emploi d'eau oxygénée et d'ammoniaque, nécessaire à la coloration d'oxydation, dégrade un peu la fibre.

D'autres systèmes ont été mis au point, notamment en utilisant des colorants portant des fonctions chimiques ayant une grande affinité chimique pour les fonctions du cheveu. Le gain en rémanence est important. Par contre, il reste insuffisant pour de nombreux utilisateurs. De plus, les fonctions chimiques du cheveu étant très dépendantes du vécu de ceux-ci, on assiste avec ce genre de système à des effets colorants très variables en fonction des cheveux sur lesquels on applique ces systèmes.

Dans le cas des traitements conditionneurs des cheveux, destinés à assouplir des cheveux jugés trop raides ou à adoucir des cheveux trop secs, les traitements actuels donnent des résultats très peu rémanents.

Il existe donc un besoin pour des compositions cosmétiques stables, en particulier pour les traitements des cheveux et qui permettent d'obtenir un effet cosmétique suffisant, en particulier pour les cheveux en mode rincé ou non rincé.

Le demandeur a remarqué, de façon surprenante, qu'il était possible de formuler des compositions cosmétiques efficaces, résistant au rinçage, en incorporant dans ces compositions des composés organiques du silicium peu ou pas polymérisés, choisis parmi les organosilanes comportant un atome de silicium et les organosiloxanes comportant deux ou trois atomes de silicium, les composés organiques du silicium comportant par molécule en outre au moins deux groupes hydroxyles ou deux groupes fonctionnels hydrolysables et au moins deux groupes fonctionnels non-hydrolysables, un au moins de ces groupes fonctionnels non-hydrolysables étant un groupe fonctionnel ayant un effet cosmétique et au moins un autre de ces groupes fonctionnels non-hydrolysables étant un groupe fonctionnel solubilisant.

Les composés organiques du silicium solubles dans l'eau, peu ou pas polymérisés sont choisis parmi les composés de formules : 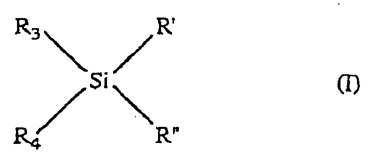 dans laquelle :
R' représente un halogène ou un groupe OR₁,
R" représente un halogène ou un groupe OR₂,
R₁ et R₂ représentent, indépendamment l'un de l'autre, l'hydrogène ou un groupe hydrocarboné saturé ou insaturé, linéaire ou ramifié,
R₃ est un groupe fonctionnel non-hydrolysable à effet cosmétique choisi parmi :
   les groupes à effet colorant nitro aromatiques, anthraquinoniques, naphtoquinoniques, benzoquinoniques, azoïques, xanthéniques, triarylméthaniques, aziniques, indoaniliques, indophénoliques ou indoaminiques,
   - les groupes à fonction anti-bactérienne de type bisguanidines, macrolides ou phénoliques,
   - les groupes à effet antifongique pyridiniques, undécyléniques, salicylés ou imidazolés, et
   - les groupes à effet réducteur, comportant une fonction thiol, acide sulfinique ou sel d'acide sulfinique, et
R₄ est un groupe fonctionnel non-hydrolysable solubilisant choisi parmi les groupes aminés, acides carboxyliques ou ses sels, acides sulfoniques ou ses sels, sulfates, ammoniums quaternaires, polyalcools, polyéthers et phosphates ;
et 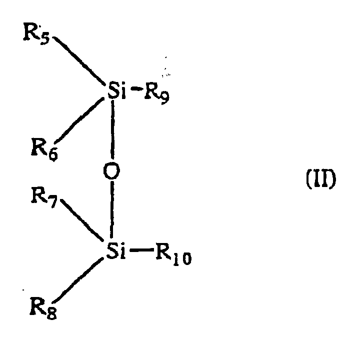 dans laquelle :
R₅ représente un groupe fonctionnel non-hydrolysable à effet cosmétique choisi parmi :
   - les groupes à effet colorant nitro aromatiques, anthraquinoniques, naphtoquinoniques, benzoquinoniques, azoïques, xanthéniques, triarytméthaniques, aziniques, indoaniliques, indophénoliques ou indoaminiques,
   - les groupes à fonction anti-bactérienne de type bisguanidines, macrolides ou phénoliques,
   - les groupes à effet antifongique pyridiniques, undécyléniques, salicylés ou imidazolés, et
   - le groupes à effet réducteur, comportant une fonction thiol, acide sulfinique ou sel d'acide sulfinique,
R₆ représente un halogène, un groupe OR'₆, R"₆, R"'₆ ou R""₆,
R₇ représente un halogène, un groupe OR'₇, R"₇, R"'₇ ou R""₇,
R₈ représente un halogène, un groupe OR'₈, R"₈, R"'₈ ou R""₈,
R₉ représente un halogène, un groupe OR'₉, R"₉, R"'₉ ou R""₉, et
R₁₀ représente un halogène, un groupe OR'₁₀, R"₁₀, R"'₁₀ ou R""₁₀,
R'₆, R"₆, R'₇, R"₇, R'₈, R"₈, R'₉, R"₉, R'₁₀ et R"₁₀ représentent, indépendarument l'un de l'autre, un groupe hydrocarboné saturé ou insaturé, linéaire ou ramifié, R'₆, R'₇, R'₈, R'₉ et R'₁₀ pouvant en outre désigner l'hydrogène,
R"'₆, R"'₇, R"'₈, R"'₉ et R"'₁₀ sont des groupes fonctionnels non-hydrolysables à effet cosmétique choisis parmi :
   - les groupes à effet colorant nitro aromatiques, anthraquinoniques, naphtoquinoniques, bentoquinoniques, azoïques, xanthéniques, triarylméthaniques, aziniques, indoaniliques, indophénoliques ou indoaminiques,
   - les groupes à fonction anti-bactérienne de type bisguanidines, macrolides ou phénoliques,
   - les groupes à effet antifongique pyridiniques, undécyléniques, salicylés ou imidazolés, et
   - les groupes à effet réducteur, comportant une fonction thiol, acide sulfinique ou sel d'acide sulfinique,
R""₆, R""₇, R""₈, R""₉ et R""₁₀ sont des groupes fonctionnels non-hydrolysables solubilisants choisis parmi les groupes aminés, acides carboxyliques ou ses sels, acides sulfoniques ou ses sels, ammoniums quaternaires, polyalcools, polyéther et phosphates ;
l'un au moins des groupes R₆ et R₉ désignant un halogène ou un groupe OR'₆ ou OR'₉,
l'un au moins des groupes R₇, R₈ et R₁₀ désignant un halogène, un groupe OR'₇, OR'₈ ou OR'₁₀, et
l'un au moins des groupes R₆, R₇, R₈, R₉ et R₁₀ désignant un groupe fonctionnel R""₆, R""₇, R""₈, R""₉ et R""₁₀. réducteurs.

Les composés organiques du silicium selon l'invention sont susceptibles de former, en milieu aqueux, un composé non hybride après condensation sur eux-mêmes, et évaporation du support. On entend par composé non hybride, un composé chimiquement homogène quant au silicium, c'est-à-dire qu'il ne renferme pas d'autres espèces métalliques ou organométalliques supplémentaires.

Les compositions selon l'invention permettent, d'une manière générale, d'obtenir des effets conditionneurs des cheveux résistant aux shampooings. En outre, la présence du groupe fonctionnel non-hydrolysable, ayant un effet cosmétique, permet d'obtenir d'autres effets cosmétiquement intéressants rémanents.

Dans la présente invention, on entend par groupe fonctionnel non-hydrolysable, ayant un effet cosmétique, des groupes chimiques fonctionnels qui, après application de la composition sur les cheveux, confèrent à ceux-ci un effet cosmétique rémanent spécifique. Ces effets cosmétiques spécifiques, sont les effets colorants, anti-bactériens ou anti-fongiques, et réducteurs.

Ainsi, par exemple, lorsque le ou les groupes fonctionnels non-hydrolysables à effet cosmétique sont des groupes à effet colorant, on peut obtenir des compositions conduisant à des effets colorants très rémanents, et ce sans utiliser d'actifs dégradants de la fibre (eau oxygénée, ammoniaque) et sans non plus se trouver confrontés à des problèmes de résultats irréguliers lorsque la composition a été appliquée sur des cheveux non uniformément sensibilisés.

Les groupes fonctionnels non-hydrolysables à effet colorant selon l'invention sont choisis parmi les groupes nitro aromatiques, anthraquinoniques, naphtoquinoniques, benzoquinoniques, azoïques, xanthéniques, triarylméthaniques, aziniques, indoaniliques, indophénoliques et indoaminiques.

Les groupes fonctionnels non-hydrolysables à effet réducteur selon l'invention sont choisis parmi les groupes comportant une fonction thiol, une fonction acide sulfinique ou sel d'acide sulfinique.

Les groupes fonctionnels non-hydrolysables à fonction anti-bactérienne selon l'invention sont choisis parmi les groupes bisguanidines, macrolides et phénoliques.

Les groupes fonctionnels non-hydrolysables à effet anti-fongique selon l'invention sont choisis parmi les groupes pyridiniques, undécyléniques, salicylés et imidazolés.

Au moins un des autres groupes fonctionnels non-hydrolysables est un groupe fonctionnel solubilisant.

On entend par groupe fonctionnel solubilisant des groupes chimiques fonctionnels facilitant la mise en solution du composé de silicium dans le solvant ou mélange de solvants de la composition, en particulier dans l'eau.

Les groupes fonctionnels non-hydrolysables, solubilisants selon l'invention sont choisis parmi les groupements d'atomes portant un radical aminé, acide carboxylique ou ses sels, acide sulfonique ou ses sels, sulfate, ammonium quaternaire, polyalcool tel que glycol, polyéther tel que polyalkylène éther et phosphate.

Parmi les fonctions aminées, on peut citer les alkylamines primaires, secondaires ou tertiaires, telles que méthylamino, alkylamino et propylamino.

Parmi les fonctions acides carboxyliques et leurs sels, on peut citer les radicaux des monoacides saturés tels que les acides acétique, propionique, butyrique, isobutyrique, valérique, isovalérique, les diacides saturés tels que les acides oxalique, malonique, succinique, glutarique et adipique, les monoacides insaturés tels que l'acide acrylique, l'acide méthacrylique, l'acide crotonique, l'acide maléique, l'acide fumarique et l'acide citraconique, les acides carbocycliques tels que l'acide benzoïque, l'acide phtalique, l'acide isophtalique, l'acide térèphtalique, les hydroxy et alcoxy-acides carboxyliques tels que l'acide glycolique, l'acide lactique, l'acide tartrique et l'acide salicylique et les sels de ces acides, en particulier les sels alcalins et plus particulièrement les sels de sodium et de potassium de ces acides.

Parmi les fonctions ammonium quaternaires, on peut citer les tétraalkylammonium, les alkylarylammonium quaternaires, les groupements alkyle et/ou aryle pouvant éventuellement comporter des fonctions telles que des fonctions acides, hydroxyles, amines et halogènes, et les ammonium quaternaires cycliques et hétérocycliques.

Parmi les acides sulfoniques et leurs sels, on peut citer les acides alkylsulfoniques tels que l'acide méthylsulfonique, les acides arylsulfoniques tels que l'acide phénylsulfonique, les acides alcoxysulfoniques tels que l'acide éthoxysulfonique, les acides alkylaryl et arylalkylsulfoniques, et les sels de ces acides, en particulier les sels alcalins de ces acides et plus particulièrement les sels de sodium et de potassium de ces acides.

Parmi les restes alkyléthers, on peut citer les poly(oxyéthylène), les poly(oxypropylène), les poly(oxytétraméthylène) et les polyglycols tels que le poly(éthylèneglycol) et le poly(propylèneglycol).

Les fonctions solubilisantes recommandées sont les fonctions amine et ammonium quaternaire.

Les groupes fonctionnels hydrolysables des composés de silicium selon l'invention sont de préférence des groupes alcoxy, aryloxy ou halogène.

Parmi les groupes alcoxy, on peut citer les groupes alkyloxy tels que méthoxy, éthoxy, propoxy et butoxy, et les groupes arylalkyloxy tels que le groupe phénylméthyloxy.

Parmi les groupes aryloxy, on peut citer le groupe phénoxy et les groupes alkylaryloxy tels que les groupes tolyloxy, éthylphényloxy et propylphényloxy.

Parmi les halogènes, on peut citer le fluor, le chlore, le brome et l'iode, le chlore étant l'halogène préféré.

De préférence, R₁ et R₂ représentent l'hydrogène, un groupe alkyle de C₁ à C₁₂, un groupe aryle de C₆ à C₁₄, un groupe alkyle de C₁ à C₈-aryle de C₆ à C₁₄, et un groupe aryle de C₆ à C₁₄-alkyle de C₁ à C₈.

Le groupe R₄ non-hydrolysable, à fonction solubilisante, est de préférence un groupe amine.

Les compositions selon l'invention contiennent des concentrations importantes en composés de silicium peu ou pas polymérisés, c'est-à-dire qu'elles contiennent au moins 0,02% en poids de composés de silicium peu ou pas polymérisés par rapport au poids total de la composition, et de préférence au moins 0,5% en poids pouvant aller jusqu'à 50% en poids.

Les concentrations en composés organiques du silicium peu ou pas polymérisés, selon l'invention, sont déterminées par des méthodes habituelles d'analyse telles que la spectroscopie RMN du silicium 29 et du proton, et par chromatographie.

Les compositions selon l'invention peuvent être des compositions aqueuses, hydroalcooliques, alcooliques, de préférence des compositions aqueuses.

Toutefois, pour des raisons de mise en oeuvre d'adjuvants, par exemple, il peut être nécessaire d'ajouter un co-solvant tel que l'alcool éthylique ou l'acétone.

De façon connue, toutes les compositions de l'invention peuvent contenir les adjuvants habituels dans le domaine cosmétique, tels que des huiles, cires ou autres corps gras usuels; des gélifiants et/ou épaississants classiques; des émulsionnants; des agents hydratants; des émollients, des filtres solaires; des actifs hydrophiles ou lipophiles comme des céramides; des agents anti-radicaux libres; des tensio-actifs; des polymères; des protéines; des bactéricides; des séquestrants; des antipelliculaires; des anti-oxydants; des conservateurs; des parfums; des charges; des matières colorantes.

Les quantités de ces différents adjuvants sont celles classiquement utilisées dans le domaine considéré.

Bien entendu, l'homme de l'art veillera à choisir le ou les éventuels composés ajoutés à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention sont utilisables en mode rincé ou non rincé.

Les compositions selon l'invention peuvent se présenter sous toutes les formes appropriées pour une application topique, notamment sous forme de solutions du type lotions ou sérum; sous forme de gels aqueux; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses.

Ces compositions sont préparées selon les méthodes usuelles.

Les compositions selon l'invention sont de préférence utilisées comme produits capillaires, notamment pour le maintien de la coiffure ou de la mise en forme des cheveux. En outre, elles peuvent apporter une coloration des cheveux, des effets anti-bactériens, anti-fongiques et réductrices.

Les compositions capillaires, selon l'invention, sont de préférence des produits de coiffage tels que des gels, des lotions de mises en pli, des lotions pour le brushing, des compositions de fixation et de coiffage telles que des laques ou spray.

Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsqu'on souhaite obtenir un spray une mousse pour la fixation ou le traitement des cheveux.

Un autre objet de l'invention est l'utilisation comme produit capillaire, de la composition cosmétique comprenant, dans un milieu cosmétiquement acceptable, au moins 0,02% en poids par rapport au poids total de la composition, d'un ou plusieurs composés organiques du silicium solubles dans l'eau, peu ou pas polymérisés, choisis parmi les composés de formules : 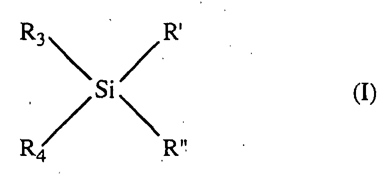 dans laquelle :
R' représente un halogène ou un groupe OR₁,
R" représente un halogène ou un groupe OR₂,
R₁ et R₂ représentent, indépendamment l'un de l'autre, l'hydrogène ou un groupe hydrocarboné saturé ou insaturé, linéaire ou ramifié,
R₃ est un groupe fonctionnel non-hydrolysable à effet cosmétique choisi parmi :
   - les groupes à effet colorant nitro aromatiques, anthraquinoniques, naphtoquinoniques, benzoquinoniques, azoïques, xanthéniques, triarylméthaniques, aziniques, indoaniliques, indophénoliques ou indoaminiques, et
   - les groupes à effet réducteur, comportant une fonction thiol, acide sulfinique ou sel d'acide sulfinique, et
R₄ est un groupe fonctionnel non-hydrolysable solubilisant choisi parmi les groupes aminés, acides carboxyliques ou ses sels, acides sulfoniques ou ses sels, sulfates, ammoniums quaternaires, polyalcools, polyéthers et phosphates ; 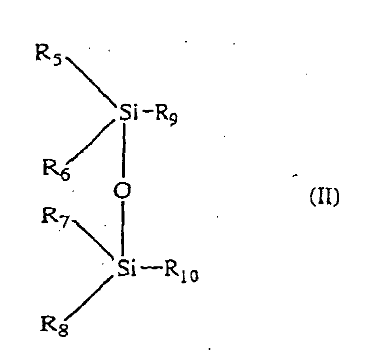
dans laquelle :
R₅ représente un groupe fonctionnel non-hydrolysable à effet cosmétique choisi parmi :
   - les groupes à effet colorant nitro aromatiques, anthraquinoniques, naphtoquinoniques, benzoquinoniques, azoïques, xanthéniques, triarylméthaniques, aziniques, indoaniliques, indophénoliques ou indoaminiques, et
   - les groupes à effet réducteur, comportant une fonction thiol, acide sulfinique ou sel d'acide sulfinique,
R₆ représente un halogène, un groupe OR'₆, R"₆, R"'₆ ou R""₆,
R₇ représente un halogène, un groupe OR'₇, R"₇, R"'₇ ou R""₇,
R₈ représente un halogène, un groupe OR'₈, R"₈, R"'₈ ou R""₈,
R₉ représente un halogène, un groupe OR'₉, R"₉, R"'₉ ou R""₉, et
R₁₀ représente un halogène, un groupe OR'₁₀, R"₁₀, R"'₁₀ au R""₁₀,
R'₆, R"₆, R'₇, R"₇, R'₈, R"₈, R'₉, R"₉, R'₁₀ et R"₁₀ représentent, indépendamment l'un de l'autre, un groupe hydrocarboné saturé ou insaturé, linéaire ou ramifié, R'₆, R'₇, R'₈, R'₉ et R'₁₀ pouvant en outre désigner l'hydrogène,
R"'₆, R"'₇, R"'₈, R"'₉ et R"'₁₀ sont des groupes fonctionnels non-hydrolysables à effet cosmétique choisis parmi :
   - les groupes à effet colorant nitro aromatiques, anthraquinoniques, naphtoquinoniques, benzoquinoniques, azoïques, xanthéniques, triarylméthaniques, aziniques, indoaniliques, indophénoliques ou indoaminiques, et
   - les groupes à effet réducteur, comportant une fonction thiol, acide sulfinique ou sel d'acide sulfinique,
R""₆, R""₇, R""₈, R""₉ et R""₁₀ sont des groupes fonctionnels non-hydrolysables solubilisants choisis parmi les groupes aminés, acides carboxyliques ou ses sels, acides sulfoniques ou ses sels, ammoniums quaternaires, polyalcools, polyéther et phosphates,
l'un au moins des groupes R₆ et R₉ désignant un halogène ou un groupe OR'₆ ou OR'₉,
l'un au moins des groupes R₇, R₈ et R₁₀ désignant un halogène, un groupe OR'₇, OR'₈ ou OR'₁₀, et
l'un au moins des groupes R₆, R₇, R₈, R₉ et R₁₀ désignant un groupe fonctionnel R""₆, R""₇, R""₈, R""₉ et R""₁₀.

La présente invention a également pour objet l'utilisation de la composition selon l'invention dans un procédé de traitement des cheveux, en vue de leur maintien et/ou coloration.

Selon un mode de réalisation de ce procédé, on applique la composition sur les cheveux rincés ou non, de préférence sous la forme d'un spray, soit à l'aide d'un flacon pompe, soit à l'aide d'un aérosol.

Après pulvérisation sur l'ensemble de la chevelure, on laisse agir et sécher la composition.

Les cheveux peuvent être mis dans la forme souhaitée, soit avant l'application, soit immédiatement après.

Le temps de séchage peut être variable et est fonction de la nature de la composition.

### EXEMPLE

On a réalisé la composition selon l'invention suivante :
- Aminopropyl-N-(4,2-dinitrophényl)amino propyl diéthoxy silane 2 g
- Mélange hydroalcoolique 50/50 98 g

On applique 10 g de la composition ci-dessus sur une perruque de 15 g de cheveux naturels, préalablement lavés, contenant 70% de cheveux blancs.

On laisse agir la composition pendant 30 minutes puis on rince à l'eau. Les cheveux sont ensuite séchés.

Les cheveux après traitement sont teints. La teinte orangée obtenue est très résistante aux shampooings. De plus, les cheveux sont très brillants et apparaissent au toucher très corporisés.

## Revendications

1. Composition cosmétique comprenant, dans un milieu cosmétiquement acceptable, au moins 0,02% en poids par rapport au poids total de la composition, d'un ou plusieurs composés organiques du silicium solubles dans l'eau, peu ou pas polymérisés, choisis parmi les composés de formules : 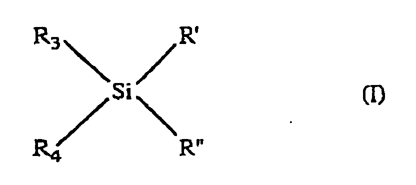 dans laquelle :
R' représente un halogène ou un groupe OR₁,
R" représente un halogène ou un groupe OR₂,
R₁ et R₂ représentent, indépendamment l'un de l'autre, l'hydrogène ou un groupe hydrocarboné saturé ou insaturé, linéaire ou ramifié,
R₃ est un groupe fonctionnel non-hydrolysable à effet cosmétique choisi parmi :
- les groupes à effet colorant nitro aromatiques, anthraquinoniques, naphtoquinoniques, benzoquinoniques, azoïques, xanthéniques, triarylméthaniques, aziniques, indoaniliques, indophénoliques ou indoaminiques,
- les groupes à fonction anti-bactérienne de type bisguanidines, macrolides ou phénoliques,
- les groupes à effet antifongique pyridiniques, undécyléniques, salicylés ou imidazolés, et
- les groupes à effet réducteur, comportant une fonction thiol, acide sulfinique ou sel d'acide sulfinique, et
R₄ est un groupe fonctionnel non-hydrolysable solubilisant choisi parmi les groupes aminés, acides carboxyliques ou ses sels, acides sulfoniques ou ses sels, sulfates, ammoniums quaternaires, polyalcools, polyéthers et phosphates ;
et 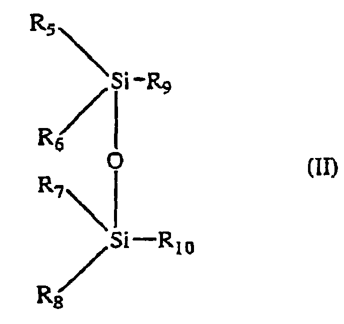 dans laquelle :
R₅ représente un groupe fonctionnel non-hydrolysable à effet cosmétique choisi parmi :
- les groupes à effet colorant nitro aromatiques, anthraquinoniques, naphtoquinoniques, benzoquinoniques, azoïques, xanthéniques, triarylméthaniques, aziniques, indoaniliques, indophénoliques ou indoaminiques,
- les groupes à fonction anti-bactérienne de type bisguanidines, macrolides ou phénoliques,
- les groupes à effet antifongique pyridiniques, undécyléniques, salicylés ou imidazolés, et
- les groupes à effet réducteur, comportant une fonction thiol, acide sulfinique ou sel d'acide sulfinique,
R₆ représente un halogène, un groupe OR'₆, R"₆, R"'₆ ou R""₆,
R₇ représente un halogène, un groupe OR'₇, R"₇, R"'₇ ou R""₇,
R₈ représente un halogène, un groupe OR'₈, R"₈, R"'₈ ou R""₈,
R₉ représente un halogène, un groupe OR'₉, R"₉, R"'₉ ou R""₉, et
R₁₀ représente un halogène, un groupe OR'₁₀, R"₁₀, R"'₁₀ ou R""₁₀,
R'₆, R"₆, R'₇, R"₇, R'₈, R"₈, R'₉, R"₉, R'₁₀ et R"₁₀ représentent, indépendamment l'un de l'autre, un groupe hydrocarboné saturé ou insaturé, linéaire ou ramifié, R'₆, R'₇, R'₈, R'₉, et R'₁₀ pouvant en outre désigner l'hydrogène,
R"'₆, R"'₇, R"'₈, R"'₉ et R"'₁₀ sont des groupes fonctionnels non-hydrolysables à effet cosmétique choisis parmi :
- les groupes à effet colorant nitro aromatiques, anthraquinoniques, naphtoquinoniques, benzoquinoniques, azoïques, xanthéniques, triarylméthaniques, aziniques, indoaniliques, indophénoliques ou indoaminiques,
- les groupes à fonction anti-bactérienne de type bisguanidines, macrolides ou phénoliques,
- les groupes à effet antifongique pyridiniques, undécyléniques, salicylés ou imidazolés, et
- les groupes à effet réducteur, comportant une fonction thiol, acide sulfinique ou sel d'acide sulfinique,
R""₆, R""₇, R""₈, R""₉ et R""₁₀ sont des groupes fonctionnels non-hydrolysables solubilisants choisis parmi les groupes aminés, acides carboxyliques ou ses sels, acides sulfoniques ou ses sels, ammoniums quaternaires, polyalcools, polyéther et phosphates ;
l'un au moins des groupes R₆ et R₉ désignant un halogène ou un groupe OR'₆ ou OR'₉,
l'un au moins des groupes R₇, R₈ et R₁₀ désignant un halogène, un groupe OR'₇, OR'₈ ou OR'₁₀, et
l'un au moins des groupes R₆, R₇, R₈, R₉ et R₁₀ désignant un groupe fonctionnel R""₆, R""₇, R""₈, R""₉, et R""₁₀.

2. Composition cosmétique selon la revendication 1, **caractérisée en ce que** les composés organiques du silicium, peu ou pas polymérisés, représentent au moins 0,5% en poids de la composition.

3. Composition selon la revendication 1 ou 2, **caractérisée en ce que** le groupe fonctionnel non-hydrolysable solubilisant est choisi parmi les groupes amines primaires, secondaires ou tertiaires.

4. Utilisation comme produit capillaire, de la composition cosmétique comprenant, dans un milieu cosmétiquement acceptable, au moins 0,02% en poids par rapport au poids total de la composition, d'un ou plusieurs composés organiques du silicium solubles dans l'eau, peu ou pas polymérisés, choisis parmi les composés de formules : 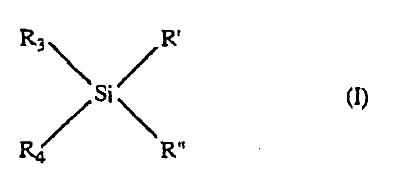 dans laquelle :
R' représente un halogène ou un groupe OR₁,
R" représente un halogène ou un groupe OR₂,
R₁ et R₂ représentent, indépendamment l'un de l'autre, l'hydrogène ou un groupe hydrocarboné saturé ou insaturé, linéaire ou ramifié,
R₃ est un groupe fonctionnel non-hydrolysable à effet cosmétique choisi parmi :
- les groupes à effet colorant nitro aromatiques, anthraquinoniques, naphtoquinoniques, benzoquinoniques, azoïques, xanthéniques, triarylméthaniques, aziniques, indoaniliques, indophénoliques ou indoaminiques, et
- les groupes à effet réducteur, comportant une fonction thiol, acide sulfinique ou sel d'acide suifinique, et
R₄ est un groupe fonctionnel non-hydrolysable solubilisant choisi parmi les groupes aminés, acides carboxyliques ou ses sels, acides sulfoniques ou ses sels, sulfates, ammoniums quaternaires, polyalcools, polyéthers et phosphates ;
et 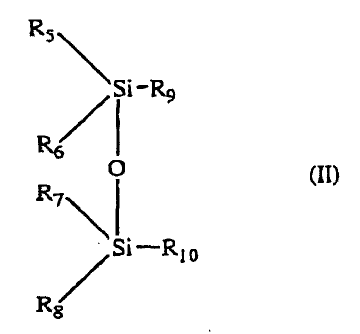 dans laquelle :
R₅ représente un groupe fonctionnel non-hydrolysable à effet cosmétique choisi parmi :
- les groupes à effet colorant nitro aromatiques, anthraquinoniques, naphtoquinoniques, benzoquinoniques, azoïques, xanthéniques, triarylméthaniques, aziniques, indoaniliques, indophénoliques ou indoaminiques, et
- les groupes à effet réducteur, comportant une fonction thiol, acide sulfinique ou sel d'acide sulfinique,
R₆ représente un halogène, un groupe OR'₆, R"₆, R"'₆ ou R""₆,
R₇ représente un halogène, un groupe OR'₇, R"₇, R"'₇ ou R""₇,
R₈ représente un halogène, un groupe OR'₈, R"₈, R"'₈ ou R""₈,
R₉ représente un halogène, un groupe OR'₉, R"₉, R"'₉ ou R""₉, et
R₁₀ représente un halogène, un groupe OR'₁₀, R"₁₀, R"'₁₀ ou R""₁₀,
R'₆, R"₆, R'₇, R"₇, R'₈, R"₈, R'₉, R"₉, R'₁₀ et R"₁₀ représentent, indépendamment l'un de l'autre, un groupe hydrocarboné saturé ou insaturé, linéaire ou ramifié, R'₆, R'₇, R'₈, R'₉ et R'₁₀ pouvant en outre désigner l'hydrogène,
R"'₆, R"'₇, R"'₈, R"'₉ et R"'₁₀ sont des groupes fonctionnels non-hydrolysables à effet cosmétique choisis parmi :
- les groupes à effet colorant nitro aromatiques, anthraquinoniques, naphtoquinoniques, benzoquinoniques, azoïques, xanthéniques, triarylméthaniques, aziniques, indoaniliquas, indophénoliques ou indoaminiques, et
- les groupes à effet réducteur, comportant une fonction thiol, acide sulfinique ou sel d'acide sulfinique,
R""₆, R""₇, R""₈, R""₉ et R""₁₀ sont des groupes fonctionnels non-hydrolysables sotubilisants choisis parmi les groupes aminés, acides carboxyliques ou ses sels, acides sulfoniques ou ses sels, ammoniums quaternaires, polyalcools, polyéther et phosphates,
l'un au moins des groupes R₆ et R₉ désignant un halogène ou un groupe OR'₆ ou OR'₉,
l'un au moins des groupes R₇, R₈ et R₁₀ désignant un halogène, un groupe OR'₇, OR'₈ ou OR'₁₀, et
l'un au moins des groupes R₆, R₇, R₈, R₉ et R₁₀ désignant un groupe fonctionnel R""₆, R""₇, R""₈, R""₉ et R""₁₀.

5. Utilisation selon la revendication 4, pour le maintien de la coiffure ou la mise en forme des cheveux.

## Patentansprüche

1. Kosmetische Zusammensetzung, die, bezogen auf das Gesamtgewicht der Zusammensetzung, mindestens 0,02 Gew.-% einer oder mehrerer, organischer, wasserlöslicher, wenig oder gar nicht polymerisierter Siliciumverbindungen, die unter den Verbindungen der folgenden Formeln ausgewählt sind, in einem kosmetisch akzeptablen Medium enthält: 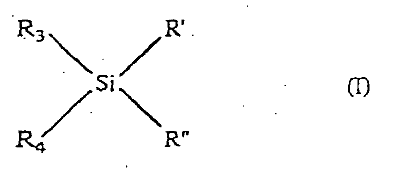 wobei in der Formel (I):
R' ein Halogen oder eine Gruppe OR₁ bedeutet,
R" ein Halogen oder eine Gruppe OR₂ bedeutet,
R₁ und R₂ unabhängig voneinander Wasserstoff oder eine geradkettige oder verzweigte, gesättigte oder ungesättigte Kohlenwasserstoffgruppe bedeuten,
R₃ eine nicht hydrolysierbare, funktionelle Gruppe mit kosmetischer Wirkung ist, die ausgewählt ist unter:
- aromatischen Nitrogruppen, Anthrachinongruppen, Naphthochinongruppen, Benzochinongruppen, Azogruppen, Xanthengruppen, Triarylmethangruppen, Azingruppen, Indoanilingruppen, Indophenolgruppen oder Indoamingruppen mit färbender Wirkung,
- Gruppen mit antibakterieller Wirkung vom Typ der Bisguanidingruppen, Macrolidgruppen oder Phenolgruppen,
- Pyridingruppen, Undecylengruppen, Salicylgruppen oder Imidazolgruppen mit fungizider Wirkung, und
- Gruppen mit reduzierender Wirkung, die eine Thiolfunktion, Sulfinsäure oder ein Sulfinsäuresalz enthalten, und
R₄ eine nicht hydrolysierbare, lösungsvermittelnde funktionelle Gruppe bedeutet, die unter den aminierten Gruppen, Carbonsäuren oder ihren Salzen, Sulfonsäuren oder ihren Salzen, Sulfaten, quartären Ammoniumgruppen, Polyalkoholen, Polyethern und Phosphaten ausgewählt ist; und 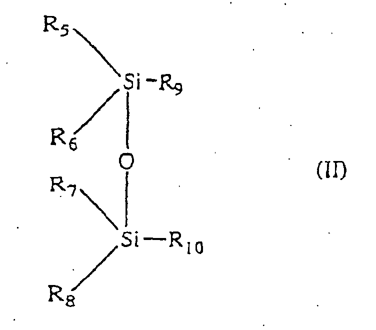
wobei in der Formel (II):
R₅ eine nicht hydrolysierbare, funktionelle Gruppe mit kosmetischer Wirkung bedeutet, die ausgewählt ist unter:
- aromatischen Nitrogruppen, Anthrachinongruppen, Naphthochinongruppen, Benzochinongruppen, Azogruppen, Xanthengruppen, Triarylmethangruppen, Azingruppen, Indoanilingruppen, Indophenolgruppen oder Indoamingruppen mit färbender Wirkung,
- Gruppen mit antibakterieller Wirkung vom Typ der Bisguanidingruppen, Macrolidgruppen oder Phenolgruppen,
- Pyridingruppen, Undecylengruppen, Salicylgruppen oder Imidazolgruppen mit fungizider Wirkung, und
- Gruppen mit reduzierender Wirkung, die eine Thiolfunktion, Sulfinsäure oder ein Sulfinsäuresalz enthalten, und
R₆ ein Halogenatom, eine Gruppe OR'₆, R''₆, R'''₆ oder R''''₆ bedeutet,
R₇ ein Halogenatom oder eine Gruppe OR'₇, R''₇, R'''₇ oder R''''₇ bedeutet,
R₈ ein Halogenatom oder eine Gruppe OR'₈, R"₈, R'"₈ oder R''''₈ bedeutet,
R₉ ein Halogenatom oder eine Gruppe OR'₉, R''₉, R'''₉ oder R''''₉, und
R₁₀ ein Halogenatom oder eine Gruppe OR'₁₀, R"₁₀, R"'₁₀ oder R""₁₀ bedeutet,
wobei die Gruppen R'₆, R"₆, R'₇, R"₇, R'₈, R''₈, R'₉, R''₉, R'₁₀ und R''₁₀ unabhängig voneinander eine gesättigte oder ungesättigte, geradkettige oder verzweigte Kohlenwasserstoffgruppe bedeuten, wobei R'₆, R'₇, R'₈, R'₉ und R'₁₀ auch Wasserstoff bedeuten können,
R'''₆, R'''₇, R'''₈, R'''₉ und R'''₁₀ nicht hydrolysierbare, funktionelle Gruppen mit kosmetischer Wirkung sind, die ausgewählt sind unter
- aromatischen Nitrogruppen, Anthrachinongruppen, Naphthochinongruppen, Benzochinongruppen, Azogruppen, Xanthengruppen, Triarylmethangruppen, Azingruppen, Indoanilingruppen, Indophenolgruppen oder Indoamingruppen mit färbender Wirkung,
- Gruppen mit antibakterieller Wirkung vom Typ der Bisguanidingruppen, Macrolidgruppen oder Phenolgruppen,
- Pyridingruppen, Undecylengruppen, Salicylgruppen oder Imidazolgruppen mit fungizider Wirkung, und
- Gruppen mit reduzierender Wirkung, die eine Thiolfunktion, Sulfinsäure oder ein Sulfinsäuresalz enthalten, und
R''''₆, R''''₇, R''''₈, R''''₉ und R''''₁₀ lösungsvermittelnde, nicht hydrolysierbare funktionelle Gruppen sind, die unter den aminierten Gruppen, Carbonsäuren oder ihren Salzen, Sulfonsäuren oder ihren Salzen, quartären Ammoniumgruppen, Polyalkoholen, Polyethern und Phosphaten ausgewählt sind;
wobei mindestens eine der Gruppen R₆ und R₉ ein Halogenatom oder eine Gruppe OR'₆ oder OR'₉ bedeutet,
mindestens eine der Gruppen R₇, R₈ und R₁₀ ein Halogenatom, eine Gruppe OR'₇, OR'₈ oder OR'₁₀ bedeutet, und
mindestens eine der Gruppen R₆, R₇, R₈, R₉ und R₁₀ eine funktionelle Gruppe R''''₆, R''''₇, R''''₈, R''''₉ und R''''₁₀ bedeutet.

2. Kosmetische Zusammensetzung nach Anspruch 1, **dadurch gekennzeichnet, dass** die wenig oder nicht polymerisierten, organischen Siliciumverbindungen mindestens 0,5 Gew.-% der Zusammensetzung ausmachen.

3. Zusammensetzung nach Anspruch 1 oder 2, **dadurch gekennzeichnet, dass** die lösungsvermittelnde, nicht hydrolisierbare, funktionelle Gruppe unter den primären, sekundären oder tertiären Aminen ausgewählt ist.

4. Verwendung einer kosmetischen Zusammensetzung, die in einem kosmetisch akzeptablen Medium, bezogen auf das Gesamtgewicht der Zusammensetzung, mindestens 0,02 Gew.-% einer oder mehrerer organischer, in Wasser löslicher, wenig oder gar nicht polymerisierter, organischer Siliciumverbindungen enthält, die unter den Verbindungen der folgenden Formeln ausgewählt sind, als Produkt für die Haarbehandlung: 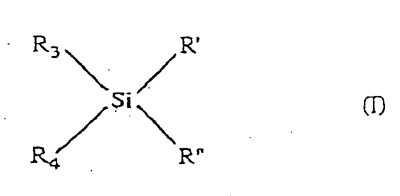 wobei in der Formel (I):
R' ein Halogen oder eine Gruppe OR₁ bedeutet,
R" ein Halogen oder eine Gruppe OR₂ bedeutet,
R₁ und R₂ unabhängig voneinander Wasserstoff oder eine geradkettige oder verzweigte, gesättigte oder ungesättigte Kohlenwasserstoffgruppe bedeuten,
R₃ eine nicht hydrolysierbare, funktionelle Gruppe mit kosmetischer Wirkung ist, die ausgewählt ist unter:
- aromatischen Nitrogruppen, Anthrachinongruppen, Naphthochinongruppen, Benzochinongruppen, Azogruppen, Xanthengruppen, Triarylmethangruppen, Azingruppen, Indoanilingruppen, Indophenolgruppen oder Indoamingruppen mit färbender Wirkung,
- Gruppen mit antibakterieller Wirkung vom Typ der Bisguanidingruppen, Macrolidgruppen oder Phenolgruppen,
- Pyridingruppen, Undecylengruppen, Salicylgruppen oder Imidazolgruppen mit fungizider Wirkung, und
- Gruppen mit reduzierender Wirkung, die eine Thiolfunktion, Sulfinsäure oder ein Sulfinsäuresalz enthalten, und
R₄ eine nicht hydrolysierbare, lösungsvermittelnde funktionelle Gruppe bedeutet, die unter den aminierten Gruppen, Carbonsäuren oder ihren Salzen, Sulfonsäuren oder ihren Salzen, Sulfaten, quartären Ammoniumgruppen, Polyalkoholen, Polyethern und Phosphaten ausgewählt ist; und 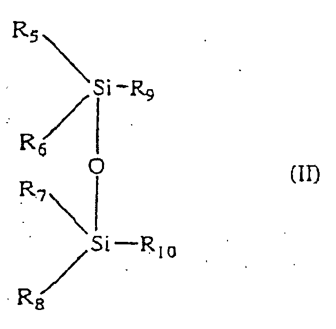
wobei in der Formel (II):
R₅ eine nicht hydrolysierbare, funktionelle Gruppe mit kosmetischer Wirkung bedeutet, die ausgewählt ist unter:
- aromatischen Nitrogruppen, Anthrachinongruppen, Naphthochinongruppen, Benzochinongruppen, Azogruppen, Xanthengruppen, Triarylmethangruppen, Azingruppen, Indoanilingruppen, Indophenolgruppen oder Indoamingruppen mit färbender Wirkung,
- Gruppen mit antibakterieller Wirkung vom Typ der Bisguanidingruppen, Macrolidgruppen oder Phenolgruppen,
- Pyridingruppen, Undecylengruppen, Salicylgruppen oder Imidazolgruppen mit fungizider Wirkung, und
- Gruppen mit reduzierender Wirkung, die eine Thiolfunktion, Sulfinsäure oder ein Sulfinsäuresalz enthalten, und
R₆ ein Halogenatom, eine Gruppe OR'₆, R''₆, R'''₆ oder R''''₆ bedeutet,
R₇ ein Halogenatom oder eine Gruppe OR'₇, R''₇, R'''₇ oder R''''₇ bedeutet,
R₈ ein Halogenatom oder eine Gruppe OR'₈, R''₈, R'''₈ oder R''''₈ bedeutet,
R₉ ein Halogenatom oder eine Gruppe OR'₉, R''₉, R'''₉ oder R''''₉, und
R₁₀ ein Halogenatom oder eine Gruppe OR'₁₀, R"₁₀, R'''₁₀ oder R''''₁₀ bedeutet,
wobei die Gruppen R'₆, R"₆, R'₇, R"₇, R'₈, R"₈, R'₉, R"₉, R'₁₀ und R"₁₀ unabhängig voneinander eine gesättigte oder ungesättigte, geradkettige oder verzweigte Kohlenwasserstoffgruppe bedeuten,
wobei R'₆, R'₇, R'₈, R'₉ und R'₁₀ auch Wasserstoff bedeuten können,
R'''₆, R'''₇, R'''₈, R'''₉ und R'''₁₀ nicht hydrolysierbare, funktionelle Gruppen mit kosmetischer Wirkung sind, die ausgewählt sind unter
- aromatischen Nitrogruppen, Anthrachinongruppen, Naphthochinongruppen, Benzochinongruppen, Azogruppen, Xanthengruppen, Triarylmethangruppen, Azingruppen, Indoanilingruppen, Indophenolgruppen oder Indoamingruppen mit färbender Wirkung,
- Gruppen mit antibakterieller Wirkung vom Typ der Bisguanidingruppen, Macrolidgruppen oder Phenolgruppen,
- Pyridingruppen, Undecylengruppen, Salicylgruppen oder Imidazolgruppen mit fungizider Wirkung, und
- Gruppen mit reduzierender Wirkung, die eine Thiolfunktion, Sulfinsäure oder ein Sulfinsäuresalz enthalten, und
R""₆, R""₇, R""₈, R""₉ und R""₁₀ lösungsvermittelnde, nicht hydrolysierbare funktionelle Gruppen sind, die unter den aminierten Gruppen, Carbonsäuren oder ihren Salzen, Sulfonsäuren oder ihren Salzen, quartären Ammoniumgruppen, Polyalkoholen, Polyethern und Phosphaten ausgewählt sind;
mindestens eine der Gruppen R₆ und R₉ ein Halogenatom oder eine Gruppe OR'₆ oder OR'₉ bedeutet,
mindestens eine der Gruppen R₇, R₈ und Rio ein Halogenatom, eine Gruppe OR'₇, OR'₈ oder OR'₁₀ bedeutet, und
mindestens eine der Gruppen R₆, R₇, R₈, R₉ und Rio eine funktionelle Gruppe R''''₆, R''''₇, R''''₈, R''''₉ und R''''₁₀ bedeutet.

5. Verwendung nach Anspruch 4, zur Festigung der Frisur oder zur Formgebung der Haare.

## Claims

1. Cosmetic composition comprising, in a cosmetically acceptable medium, at least 0.02% by weight, relative to the total weight of the composition, of one or more water-soluble, unpolymerized or relatively unpolymerized organosilicon compounds chosen from the compounds of formulae: 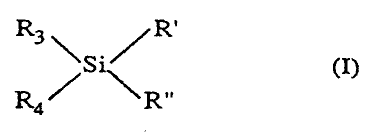 in which:
R' represents a halogen or a group OR₁,
R" represents a halogen or a group OR₂,
R₁ and R₂ represent, independently of each other, hydrogen or a saturated or unsaturated, linear or branched hydrocarbon-based group,
R₃ is a non-hydrolysable functional group with a cosmetic effect chosen from:
- nitro-aromaticgroups, anthraquinone groups, naphthoquinone groups, benzoquinone groups, azo groups, xanthene groups, triarylmethane groups, azine groups, indoaniline groups, indophenol groups or indoamine groups with a colouring effect,
- groups with an antibacterial function of the bisguanidine, macrolide or phenolic type,
- pyridine groups, undecylenic groups, salicyl groups or imidazole groups with an antifungal effect, and
- groups having a reducing effect, comprising a thiol, sulfinic acid or sulfinic acid salt functional group, and
R₄ is a solubilizing non-hydrolysable functional group chosen from amine groups, carboxylic acids or salts thereof, sulfonic acids or salts thereof, sulfates, quaternary ammoniums, polyalcohols, polyethers and phosphates; and 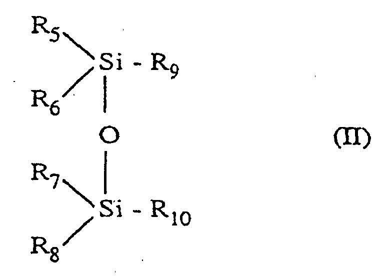
in which:
R₅ represents a non-hydrolysable functional group with a cosmetic effect chosen from:
- nitroaromatic groups, anthraquinone groups, naphthoquinone groups, benzoquinone groups, azo groups, xanthene groups, triarylmethane groups, azine groups, indoaniline groups, indophenol groups or indoamine groups with a colouring effect,
- groups with an antibacterial function of the bisguanidine, macrolide or phenolic type,
- pyridine groups, undecylenic groups, salicyl groups or imidazole groups with an antifungal effect, and
- groups having a reducing effect, comprising a thiol, sulfinic acid or sulfinic acid salt functional group,
R₆ represents a halogen or a group OR'₆, R"₆, R"'₆ or R""₆,
R₇ represents a halogen or a group OR'₇, R"₇, R"'₇ or R""₇,
R₈ represents a halogen or a group OR'₈, R"₈, R"'₈ or R""₈,
R₉ represents a halogen or a group OR'₉, R"₉, R"'₉ or R""₉, and
R₁₀ represents a halogen or a group OR'₁₀, R"₁₀, R"'₁₀ or R""₁₀,
R'₆, R"₆, R'₇, R"₇, R'₈, R"₈, R'₉, R"₉, R'₁₀ and R"₁₀ represent, independently of each other, a saturated or unsaturated, linear or branched hydrocarbon-based group, R'₆, R'₇, R'₈, R'₉ and R'₁₀ also possibly denoting hydrogen;
R"'₆, R"'₇, R"'₈, R"'₉ and R"'₁₀ are non-hydrolysable functional groups with a cosmetic effect chosen from:
- nitro-aromaticgroups, anthraquinone groups, naphthoquinone groups, benzoquinone groups, azo groups, xanthene groups, triarylmethane groups, azine groups, indoaniline groups, indophenol groups or indoamine groups with a colouring effect,
- groups with an antibacterial function of the bisguanidine, macrolide or phenolic type,
- pyridine groups, undecylenic groups, salicyl groups or imidazole groups with an antifungal effect, and
- groups having a reducing effect, comprising a thiol, sulfinic acid or sulfinic acid salt functional group,
R""₆, R""₇, R""₈, R""₉ and R""₁₀ are solubilizing non-hydrolysable functional groups chosen from amine groups, carboxylic acids or salts thereof, sulfonic acids or salts thereof, quaternary ammoniums, polyalcohols, polyethers and phosphates;
at least one of the groups R₆ and R₉ denoting a halogen or a group OR'₆ or OR'₉,
at least one of the groups R₇, R₈ and R₁₀ denoting a halogen or a group OR'₇, OR'₈ or OR'₁₀, and
at least one of the groups R₆, R₇, R₈, R₉ and R₁₀ denoting a functional group R""₆, R""₇, R""₈, R""₉ and R""₁₀.

2. Cosmetic composition according to Claim 1, **characterized in that** the unpolymerized or relatively unpolymerized organosilicon compounds represent at least 0.5% by weight of the composition.

3. Composition according to either of Claims 1 and 2, **characterized in that** the non-hydrolysable solubilizing functional group is chosen from primary, secondary and tertiary amine groups.

4. Use, as a hair product, of the cosmetic composition comprising, in a cosmetically acceptable medium, at least 0.02% by weight, relative to the total weight of the composition, of one or more water-soluble, unpolymerized or sparingly polymerized organosilicon compounds chosen from the compounds of formulae: 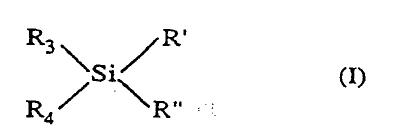 in which:
R' represents a halogen or a group OR₁,
R" represents a halogen or a group OR₂'
R₁ and R₂ represent, independently of each other, hydrogen or a saturated or unsaturated, linear or branched hydrocarbon-based group,
R₃ is a non-hydrolysable functional group with a cosmetic effect chosen from:
- nitro-aromatic groups, anthraquinone groups, naphthoquinone groups, benzoquinone groups, azo groups, xanthene groups, triarylmethane groups, azine groups, indoaniline groups, indophenol groups or indoamine groups with a colouring effect, and
- groups with a reducing effect, comprising a thiol, sulfinic acid or sulfinic acid salt functional group, and
R₄ is a solubilizing non-hydrolysable functional group chosen from amine groups, carboxylic acids or salts thereof, sulfonic acids or salts thereof, sulfates, quaternary ammoniums, polyalcohols, polyethers and phosphates;
and 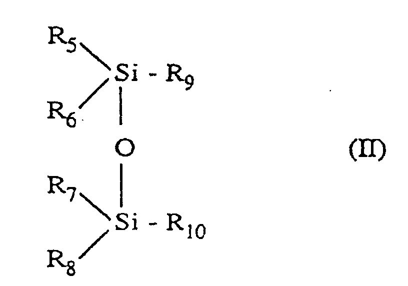 in which:
R₅ represents a non-hydrolysable functional group with a cosmetic effect chosen from:
- nitro-aromatic groups, anthraquinone groups, naphthoquinone groups, benzoquinone groups, azo groups, xanthene groups, triarylmethane groups, azine groups, indoaniline groups, indophenol groups or indoamine groups with a colouring effect, and
- groups with a reducing effect, comprising a thiol, sulfinic acid or sulfonic acid salt functional group,
R₆ represents a halogen or a group OR'₆, R"₆, R"'₆ or R""₆,
R₇ represents a halogen or a group OR'₇, R"₇, R"'₇ or R""₇,
R₈ represents a halogen or a group OR'₈, R"₈, R"'₈ or R""₈,
R₉ represents a halogen or a group OR'₉, R"₉, R"'₉ or R""₉, and
R₁₀ represents a halogen or a group OR'₁₀, R"₁₀, R"'₁₀ or R""₁₀,
R'₆, R"₆, R'₇, R"₇, R'₈, R"₈, R'₉, R"₉, R'₁₀ and R"₁₀ represent, independently of each other, a saturated or unsaturated, linear or branched hydrocarbon-based group, R'₆, R'₇, R'₈, R'₉ and R'₁₀ also possibly denoting hydrogen:
R"'₆, R"'₇, R"'₈, R"'₉ and R"'₁₀ are non-hydrolysable functional groups with a cosmetic effect chosen from:
- nitro-aromatic groups, anthraquinone groups, naphthoquinone groups, benzoquinone groups, azo groups, xanthene groups, triarylmethane groups, azine groups, indoaniline groups, indophenol groups or indoamine groups with a colouring effect, and
- groups with a reducing effect, comprising a thiol, sulfinic acid or sulfinic acid salt functional group,
R""₆, R""₇, R""₈, R""₉ and R""₁₀ are solubilizing non-hydrolysable functional groups chosen from amine groups, carboxylic acids or salts thereof, sulfonic acids or salts thereof, quaternary ammoniums, polyalcohols, polyethers and phosphates,
at least one of the groups R₆ and R₉ denoting a halogen or a group OR'₆ or OR'₉,
at least one of the groups R₇, R₈ and R₁₀ denoting a halogen or a group OR'₇, OR'₈ or, OR'₁₀, and
at least one of the groups R₆, R₇, R₈, R₉ and R₁₀ denoting a functional group R""₆, R""₇, R""₈, R""₉ or R""₁₀.

5. Use according to Claim 4, for holding the hairstyle or for shaping the hair.
